# EUROPEAN PATENT APPLICATION

(11) **EP 2 803 969 A1**
(43) Date of publication of application: **19.11.2014**
(21) Application number: 14001730.2
(22) Date of filing: 15.05.2014
(51) Int. Cl.: G01N 1/22, G01N 1/26, G01N 33/00, G01M 15/10

(54) **Exhaust Gas Analysis System, Exhaust Gas Collecting Device and Exhaust Gas Leak Detecting Method**

(30) Priority: 17.05.2013 JP 2013104753
(71) Applicant: Public Interest Incorporated Foundation Japan Automobile Transport Technology Association, Tokyo 102-0085 (JP); HORIBA, LTD., Kyoto-shi, Kyoto 601-8510 (JP)
(72) Inventor: Noda, Akira, Tokyo, 102-0085 (JP); Hara, Ippei, Tokyo, 102-0085 (JP); Yokoyama, Hideya, Tokyo, 102-0085 (JP); Higuchi, Masahiro, Kyoto-shi, Kyoto, 601-8510 (JP); Yoshimura, Sayaka, Kyoto-shi, Kyoto, 601-8510 (JP)
(74) Representative: Müller - Hoffmann & Partner

(57) **Abstract**

An exhaust gas analysis system of the present invention includes: an exhaust gas collecting device for collecting exhaust gas from an exhaust pipe; and exhaust gas analysis equipment for analyzing the exhaust gas. The exhaust gas collecting device includes: an exhaust gas collecting part which is provided at an exhaust gas discharge port of the exhaust pipe, having an exhaust gas collecting port that is larger than the exhaust gas discharge port, for collecting air around the exhaust gas discharge port together with the exhaust gas; a main flow passage which is connected to the exhaust gas collecting part and in which the exhaust gas and the air collected from the exhaust gas collecting part flow; and an exhaust gas detecting mechanism for continuously detecting a predetermined component contained in the exhaust gas in the vicinity of the exhaust gas collecting port in the exhaust gas collecting part.

## Description

### Technical Field

The present invention relates to an exhaust gas analysis system and an exhaust gas collecting device for collecting exhaust gas discharged from an internal combustion engine or an exhaust gas leak detecting method for detecting a leak of exhaust gas discharged from an internal combustion engine.

### Background Art

As a device for measuring a concentration of a substance contained in exhaust gas of an internal combustion engine, there is an exhaust gas analysis device including an open type exhaust gas collecting part (referred to as "open type collecting part" hereinafter) as disclosed in, for example, Patent Literature 1.

This exhaust gas analysis device includes: an open type collecting part for sucking exhaust gas discharged from an exhaust pipe of a vehicle and sucking air around the exhaust pipe; a flow passage in which mixed gas obtained by mixing the exhaust gas and the air sucked from this open type collecting part flows; and a measuring part installed in this flow passage and measuring a concentration of a predetermined measurement component contained in the mixed gas.

### Citation List

### Patent Literature

Patent Literature 1: U.S. Pat. No. 5907109

### Summary of Invention

### Technical Problem

However, in the exhaust gas analysis device equipped with an open type collecting part, since there is a space sucking air between the open type collecting part and the exhaust pipe, there is a possibility that the exhaust gas leaks to the outside from this space and there is a risk that the exhaust gas analysis cannot be performed accurately to be problematic.

Further, in the Specific Notice Attachment 44 "Measurement Method for Motorcycle Exhaust Gas" for Road Vehicle Safety Criteria describing test procedures for an exhaust gas test or an international standards, it is defined to collect exhaust gas without leak exhaust gas discharged from an internal combustion engine and it is necessary to suppress a leak of the exhaust gas.

However, in the conventional open type collecting part disclosed in Patent Literature 1, since it is not possible to confirm whether or not the exhaust gas leaks, it is not known whether the exhaust gas discharged from the internal combustion engine can be collected by the open type collecting part without a leak and therefore it is not possible to ensure reliability for the test results of the exhaust gas test.

As a method of solving this problem, it can be considered to suppress a leak of the exhaust gas by inserting the exhaust pipe to the open type collecting part. However, in the case where the exhaust pipe is excessively inserted to the open type collecting part, an extra load is applied to the internal combustion engine and test conditions are changed, and it becomes difficult to accurately perform the exhaust gas analysis.

Meanwhile, in the case where the space between the open type exhaust gas collecting part and the exhaust pipe is excessively large, there is a possibility that the exhaust gas leaks from the space between the open type exhaust gas collecting part and the exhaust pipe, and these methods can not be a solution of the above problems.

### Summary of the Invention

Therefore, the present invention has been made in view of the above problems, and an essential object thereof is to detect exhaust gas leaking from a space between an exhaust pipe and an open type exhaust gas collecting part in an exhaust gas collecting device using the open type exhaust gas collecting part. Solution to Problem

That is, in one aspect of the present invention, an exhaust gas analysis system includes: an exhaust gas collecting device for collecting exhaust gas from an exhaust pipe in which the exhaust gas discharged from an internal combustion engine flows; and gas analysis equipment for analyzing the exhaust gas collected by the exhaust gas collecting device. In this configuration, it is characterized that the exhaust gas collecting device includes: an exhaust gas collecting part which is provided at an exhaust gas discharge port of the exhaust pipe, having an exhaust gas collecting port that is larger than the exhaust gas discharge port, for collecting air around the exhaust gas discharge port together with the exhaust gas discharged from the exhaust gas discharge port; a main flow passage which is connected to the exhaust gas collecting part and in which the exhaust gas and the air collected from the exhaust gas collecting part flow; and an exhaust gas detecting mechanism for continuously detecting a predetermined component contained in the exhaust gas in the vicinity of the exhaust gas collecting port in the exhaust gas collecting part.

With this configuration, since the exhaust gas detecting mechanism is provided, it is possible to detect whether the exhaust gas is leaked between the exhaust pipe and the exhaust gas collecting part by confirming the detection result of the exhaust gas detecting mechanism. Moreover, since the exhaust gas detecting mechanism is provided in the vicinity of the exhaust gas collecting port, it is possible to prevent that it becomes difficult for the exhaust gas detecting mechanism to detect the predetermined component contained in the exhaust gas because of excessive dilution of the leaked exhaust gas with the air around the exhaust pipe.

Thus, it is possible to accurately perform the exhaust gas analysis using an open type exhaust gas collecting part. Further, since it becomes possible to perform the test in conformance with a measuring method defined in such as the Road Vehicle Safety Standard or the international standards, it is possible to ensure reliability for the test result of the exhaust gas test.

Furthermore, since the exhaust gas detecting mechanism is provided for continuously detecting the predetermined component, the leak of the exhaust gas can be detected in real time.

Further, it is preferable that the exhaust gas detecting mechanism includes: a detection sampling part for collecting gas in the vicinity of the exhaust gas collecting port of the exhaust gas collecting part; and a leak detecting part for detecting a concentration of the predetermined component contained in the exhaust gas in the gas collected by the detection sampling part, and that the detection sampling part includes a plurality of sampling pipes in the vicinity of the exhaust gas collecting port. Thus, by sampling the gas at a plurality of points in the vicinity of the exhaust gas collecting port, it is possible to perform the sampling of the gas accurately to detect the leak of the exhaust gas even in the case where s leaking location cannot be determined due to such as vibrations of the exhaust pipe.

In this configuration, it is preferable that the exhaust gas detecting mechanism is adapted to measure carbon dioxide or carbon monoxide. Since the carbon dioxide or carbon monoxide contained in the exhaust gas is higher in concentration compared to carbon dioxide or carbon monoxide contained in the air and exhibits a large difference in concentration, it becomes easy to detect the leak of the exhaust gas.

In this configuration, in the case where the exhaust gas collecting port of the exhaust gas collecting part is larger than an opening in cross section of a connecting portion connected to the exhaust gas collecting part in the main flow passage, a throttle surface for connecting between the exhaust gas collecting port and the opening in cross section of the main flow passage is to be provided in an inner peripheral surface of the exhaust gas collecting part.

In the case where this throttle surface has a flat surface facing the exhaust gas collecting port, there is a possibility that the exhaust gas and air discharged from the exhaust pipe toward the main flow passage may rebound on this flat surface and leak to the outside from a portion between the exhaust gas collecting part and the exhaust pipe.

Therefore, as a specific aspect of the exhaust gas collecting part in the present invention, it is preferable that the exhaust gas collecting part has a taper surface with an opening in cross section thereof gradually decreases toward a side of the connecting portion of the main flow passage from a side of the exhaust gas collecting port in the inner peripheral surface of the exhaust gas collecting part.

With this configuration, even in the case where the exhaust gas and air discharged from the exhaust pipe toward the main flow passage rebound on the taper surface, it is possible to facilitate the exhaust gas and air to flow into the main flow passage without leaking to the outside from the portion between the exhaust gas collecting part and the exhaust pipe.

It is preferable that, the exhaust gas analysis system of the present invention further includes a constant flow mechanism provided on the main flow passage for maintaining a flow rate of fluid flowing in the main flow passage constant and further includes a dilution gas flow passage provided on the main flow passage and connected to an upstream side of the constant flow mechanism for introducing dilution gas into the main flow passage.

With this configuration, the flow rate of the air collected from the exhaust gas collecting part can be adjusted by adjusting the flow rate of the dilution gas introduced from the dilution gas flow passage. For example, in the case where a user determines that the exhaust gas is leaking based on a detected result by the exhaust gas detecting mechanism, the flow rate of the air to be collected by the exhaust gas collecting part can be increased by decreasing the flow rate of the dilution gas flowing in the dilution gas introducing flow passage, and thus the leak of the exhaust gas can be eliminated. Moreover, since the leak of the exhaust gas can be eliminated without changing the flow rate of the fluid flowing in the main flow passage by the constant flow mechanism, it is possible to accurately perform the exhaust gas analysis without largely varying a dilution ratio of the exhaust gas.

In another aspect of the present invention, an exhaust gas leak detecting method of an exhaust gas analysis system includes exhaust gas analysis equipment for analyzing exhaust gas collected by an exhaust gas collecting device for collecting exhaust gas from an exhaust pipe in which the exhaust gas discharged from an internal combustion engine flows. In this method, it is characterized that, the exhaust gas collecting device includes: an exhaust gas collecting part which is provided at an exhaust gas discharge port of the exhaust pipe in which the exhaust gas flows and has an exhaust gas collecting port that is larger than the exhaust gas discharge port, and an exhaust gas detecting mechanism for detecting a predetermined component contained in the exhaust gas in the vicinity of the exhaust gas collecting port in the exhaust gas collecting part, thereby detecting a leak of the exhaust gas using detection results detected by the exhaust gas detecting mechanism.

With this method, in the case where the leak of the exhaust gas to the outside of the exhaust gas collecting part is detected, for example, positions of the exhaust gas discharge port of the exhaust pipe and the exhaust gas collecting port of the exhaust gas collecting part are adjusted to take actions for eliminating the leak of the exhaust gas and the exhaust gas analysis can be accurately performed. Further, since it becomes possible to perform the test in conformance with a measuring method defined in such as the Road Vehicle Safety Standard or the international standards, it is possible to ensure reliability for the test result of the exhaust gas test.

### Advantageous Effects of Invention

According to the present invention configured as described above, it is possible to detect the exhaust gas leaking from a portion between the exhaust pipe and the open type exhaust gas collecting part in collecting the exhaust gas using the open type exhaust gas collecting part.

### Brief Description of Drawings

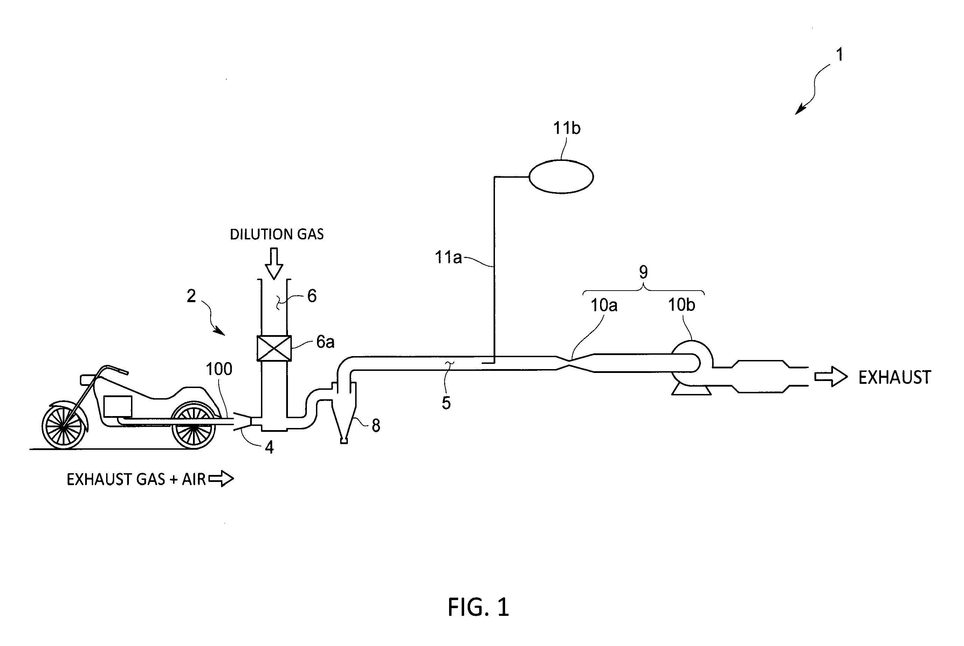

Fig. 1 is a schematic diagram showing an exhaust gas collecting system in the present embodiment;
Fig. 2 is a side view showing an exhaust gas collecting part in the present embodiment;
Fig. 3 is a cross-sectional view of the exhaust gas collecting part in the present embodiment;
Fig. 4 is a front view showing the exhaust gas collecting part in the present embodiment;
Fig. 5 is a graph showing a CO concentration measured by an exhaust gas detecting mechanism when a test motorcycle was test-travelled;
Fig. 6 is a graph showing a CO₂ concentration measured by the exhaust gas detecting mechanism when a test motorcycle was test-travelled; and
Fig. 7 is a front view showing an exhaust gas collecting part in another embodiment.

### Description of Embodiments

The following describes one embodiment of an exhaust gas analysis system using an exhaust gas collecting device pertaining to the present invention with reference to the accompanying drawings.

The exhaust gas analysis system 1 in the present embodiment is a quantitative dilution analysis device (open type CVS) provided with an open type exhaust gas collecting device. This exhaust gas analysis system 1 is adapted to collect exhaust gas discharged from an exhaust pipe 100 of an internal combustion engine, for example, in an automobile or a motorcycle and dilute the collected exhaust gas with dilution gas such as air, and then a concentration of a predetermined component of the diluted exhaust gas is to be detected.

The exhaust gas analysis system 1 of the present embodiment includes: an open type exhaust gas collecting part 4 for collecting the exhaust gas discharged from the exhaust pipe 100 and air around the exhaust pipe 100; and a main flow passage 5 which is connected to the exhaust gas collecting part 4 so that the exhaust gas and air collected from the exhaust gas collecting part 4 flow therein.

The exhaust gas collecting part 4 is intended to be provided so as to be substantially opposite to an exhaust gas discharge port 100a of the exhaust pipe 100. This exhaust gas collecting part 4 has an exhaust gas collecting port 4a which is larger than the exhaust gas discharge port 100a and it is intended to collect the air around the exhaust gas discharge port 100a together with the exhaust gas discharged from the exhaust gas discharge port 100a.

The exhaust gas collecting part 4 has a hollow circular tube shape and it includes the exhaust gas collecting port 4a having a circular open shape which is formed in an opening part 40 in an upstream side. This exhaust gas collecting port 4a is arranged so as to be substantially concentric with the exhaust gas discharge port 100a of the exhaust pipe 100. Further, the main flow passage 5 is connected to an opening part in a downstream side of the exhaust gas collecting part 4.

Further, an inner peripheral surface of the exhaust gas collecting part 4 is formed as a taper surface 4b having an opening in cross section gradually decreasing from the exhaust gas collecting port 4a toward a connecting portion 4c which is connected to the main flow passage 5. In the present embodiment, by forming the exhaust gas collecting part 4 to have a truncated cone tubular shape, the inner peripheral surface thereof is formed as the taper surface 4b.

The main flow passage 5 has a dilution gas introducing flow passage 6 for introducing the dilution gas into the main flow passage 5; a mixing part 8 for stirring the exhaust gas, air and dilution gas to be mixed; and a constant flow mechanism 9 for keeping the flow rate of the fluid flowing in the main flow passage 5 constant, and these parts 6, 8 and 9 are arranged from the upstream side in this order.

The dilution gas introducing flow passage 6 is connected to the main flow passage 5 for allowing the dilution gas such as air to flow into the main flow passage 5. A flow adjusting valve 6a is provided on the dilution gas introducing flow passage 6 in order that, for example, a user can appropriately vary a flow rate of the dilution gas.

The mixing part 8 includes a cyclone which is provided in a downstream side of a confluence of the main flow passage 5 and the dilution gas introducing flow passage 6, and this cyclone is adapted to remove dusts and stir the exhaust gas and air to be mixed to thereby produce the mixed gas obtained by diluting the exhaust gas with the air. Here, the air to be stirred and mixed with the exhaust gas by the mixing part 8 is the air collected by the exhaust gas collecting part 4 and the air that is the dilution gas introduced from the dilution gas introducing flow passage 6.

The constant flow mechanism 9 is intended to control the flow rate so that the total flow rate of the mixed gas becomes constant flow rate and it is configured of a venture 10a that is composed of a critical flow venture (CFV) and a suction pump 10b such as a blower that is connected to a downstream of this venture 10a.

Then, the mixed gas is sucked by the suction pump 10b and a pressure difference between the upstream side and the downstream side of the venture 10a is set to be a predetermined value or higher. Thus, the total flow rate of the mixed gas flowing in the main flow passage 5 is kept to be constant. Here, the mixed gas sucked by the suction pump 10b is discharged to the outside.

Here, in the present embodiment, a sampling line 11a is connected to a portion between the mixing part 8 and the constant flow mechanism 9 in the main flow passage 5 for collecting a part of the mixed gas flowing in the main flow passage 5. Analysis equipment 11b is connected to this sampling line 11a for analyzing the mixed gas collected through the sampling line 11a.

The analysis equipment 11b includes, for example, a collecting bag for accumulating the collected mixed gas. A concentration of a predetermined component contained in the mixed gas accumulated in this collecting bag is analyzed by an analyzer such as, for example, NDIR (non-dispersive infrared analyzer).

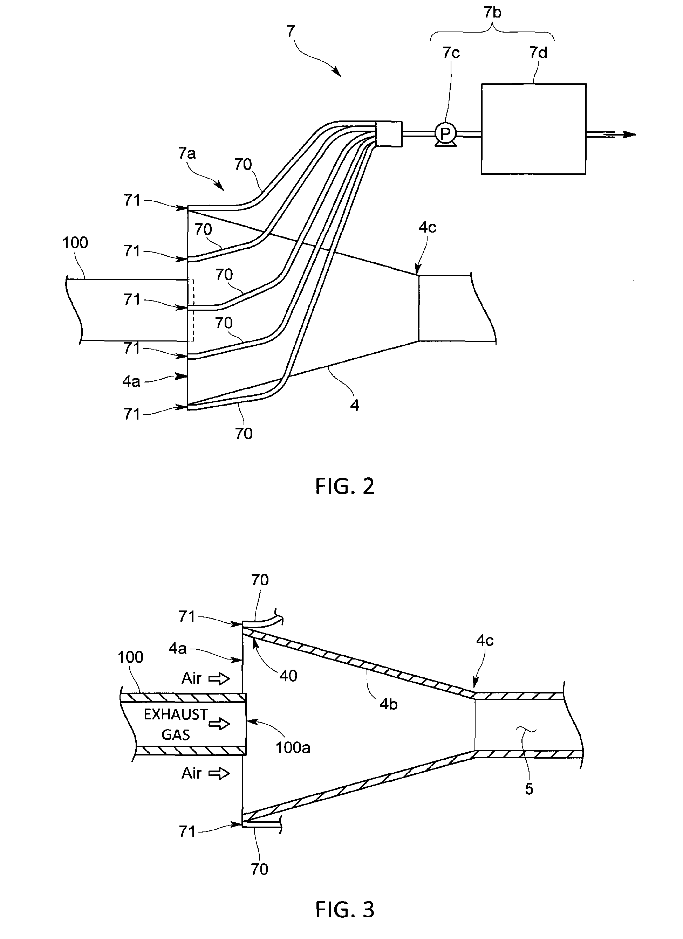

Thus, as shown in Figs. 2 to 4, the exhaust gas collecting part 4 of the present embodiment is provided with an exhaust gas detecting mechanism 7 for detecting a predetermined component contained in the exhaust gas for detecting a leak of the exhaust gas.

The exhaust gas detecting mechanism 7 includes: a detection sampling part 7a for collecting gas in the vicinity of the exhaust gas collecting port 4a of the exhaust gas collecting part 4; and a leak detecting part 7b for detecting a concentration of a predetermined component contained in the exhaust gas in the gas collected by the detection sampling part 7a. Further, the leak detecting part 7b includes: a pump 7c for sampling the gas; and a detector 7d for detecting the gas component.

In this context, the term "vicinity" implies any position where the exhaust gas leaking from the exhaust gas collecting port 4a can be collected in the exhaust gas collecting part 4, and, for example, an inner peripheral surface, an outer peripheral surface or a distal end surface of the opening portion 40 of the exhaust gas collecting part 4 where the exhaust gas collecting port 4a is formed, may be considered.

The detection sampling part 7a is opened in the vicinity of the exhaust gas collecting port 4a and includes, for example, a flexible sampling tube 70. In specific, the sampling tube 70 has an opening end portion 71 forming an upstream side opening (sampling opening) that is provided on an outer peripheral surface of the opening portion 40 of the exhaust gas collecting part 4. In the present embodiment, an opening direction of the upstream side opening end portion 71 of the sampling tube 70 is the same direction as an opening direction of the exhaust gas collecting part 4 or an inclination direction of the taper surface 4b of the exhaust gas collecting part 4. Here, the detection sampling part 7a samples the gas in the vicinity of the exhaust gas collecting port 4a by the sampling tube 70, the gas component detector 7d or the pump 7c which is provided in the upstream or downstream of the gas component detector 7d.

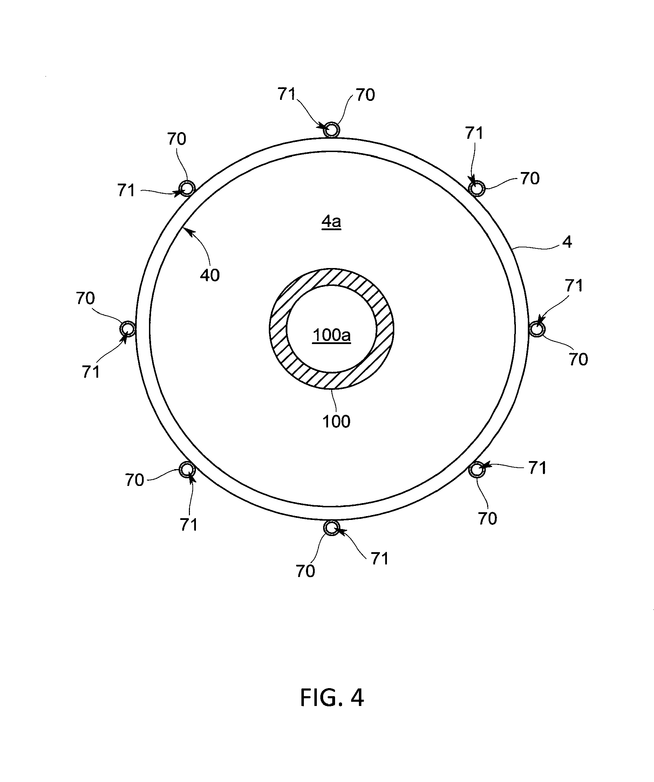

The detection sampling part 7a of the present embodiment includes multiple sampling tubes 70 (i.e., eight tubes in Fig. 4) and the opening end portions 71 of the multiple sampling tubes 70 are provided at equal intervals in a circumferential direction so as to surround the opening portion 40 of the exhaust gas collecting port 4a. With this configuration, even in the case where relative positions at the time of setting or positions of the exhaust gas discharge port 100a and the exhaust gas collecting port 4a are shifted due to vibration of the exhaust pipe 100, the exhaust gas can be collected without a leak. Further, one ends in the downstream side of the sampling tubes 70 are bundled to be a single tube and connected to the leak detecting part 7b. Note that the number of the sampling tubes 70 is appropriately changed according to situations.

The leak detecting part 7b is adapted to continuously measure a concentration of a predetermined component contained in the exhaust gas in the gas collected by the detection sampling part 7a. Specifically, the leak detecting part 7b is composed of an exhaust gas analysis device that detects the concentration of the predetermined component by a non-dispersive infrared-ray absorbing method of applying, for example, infrared light emitted from an infrared light source to the gas collected from the detection sampling part 7a and using property of absorbing this infrared light by the predetermined component contained in the gas. Further, the leak detecting part 7b outputs the measured concentration to a display part such as a monitor.

Then, a user can detect whether the exhaust gas leaks from the exhaust gas collecting port 4a by comparing the measured concentration outputted to this display part to a predetermined threshold.

Here, various components such as CO, CO₂ or NOx contained in the exhaust gas can be used as the predetermined component to be detected by the leak detecting part 7b, whereas CO₂ is desirable. This reason is shown along with the following test results.

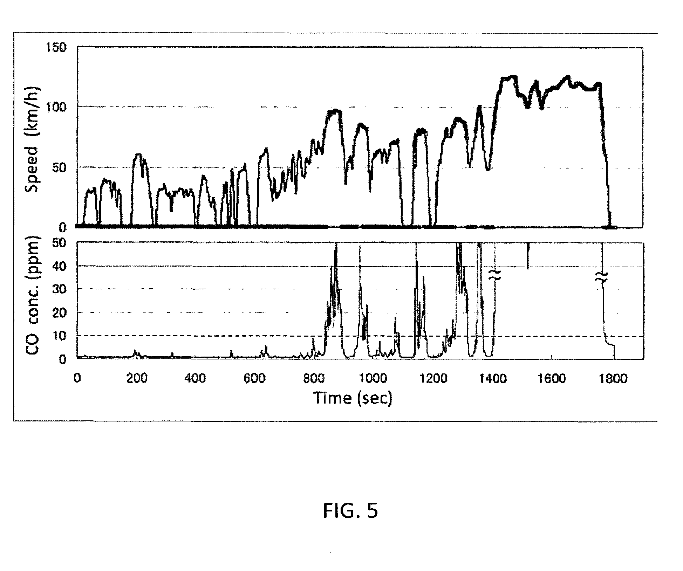

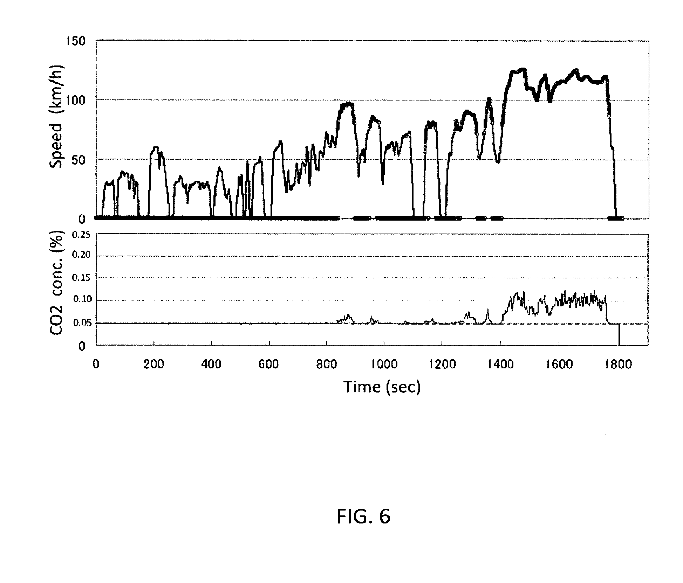

Figs. 5 and 6 are test results showing the detected concentrations of CO gas and CO₂ gas when driving a test motorcycle in WMTC (Worldwide-harmonized Motorcycle Test Cycle) mode that is used in an official exhaust gas test where the discharge port 100a of the exhaust pipe 100 of the test motorcycle and the exhaust gas collecting port 4a of the exhaust gas collecting part 4 are disposed with a space of 5 cm while setting the suction quantity Q_{FNL} of the suction pump 10b disposed in the main flow passage 5 to be 4 m³/min.

Here, Fig. 5 is the test result when the leak detecting part 7b detects CO as the predetermined component, and Fig. 6 is the test result when the leak detecting part 7b detects CO₂ as the predetermined component.

It is noted that, in this test, CO is set to 10 ppm and CO₂ is set to 0.05% tentatively as the threshold for leak determination and it is determined that there is a possibility of the exhaust gas leaking when the measured concentration exceeds this threshold value.

In Figs. 5 and 6, when accelerating at a vehicle speed of 50 km/h or more, the CO concentration exceeds the threshold 10 ppm and the CO₂ concentration exceeds the threshold 0.05 %, and therefore the both test results show that there is a high possibility of the exhaust gas leaking.

Here, significant peaks were observed in the CO concentration shown in Fig. 5 than in the CO₂ concentration shown in Fig. 6. This reason is considered that, since CO is a component that is hardly contained in the air unlike CO₂, and in the case where the exhaust gas discharged from the test motorcycle leaks and is collected by the detection sampling part 7a, the leak detecting part 7b detects CO contained in this exhaust gas.

Therefore, although CO seems to be suitable as a leak sensing gas, in the case of a vehicle equipped with a three-way catalyst system that purifies harmful components in the exhaust gas by reduction / oxidation, the CO concentration is significantly lowered and such a significant peak could not be obtained.

Meanwhile, since CO₂ contained in the exhaust gas discharged from the motorcycle would be mixed with CO₂ present in the air, there is no significant peak compared to CO, whereas the exhaust gas leak could be detected (see Fig. 6).

Therefore, it is considered desirable to use CO₂ as the predetermined component to be detected by the leak detecting part 7b of the exhaust gas detecting mechanism 7 in view of being able to detect a leak even in the case of a vehicle equipped with a three-way catalyst system.

Next, the following describes a method of detecting an exhaust gas leak in the present embodiment.

First, after the exhaust gas collecting port 4a of the exhaust gas collecting part 4 is disposed in a predetermined collecting position with respect to the exhaust gas discharge port 100a of the exhaust pipe 100, the internal combustion engine is started. Then, upon starting the suction pump 10b of the exhaust gas analysis system 1, the exhaust gas collecting port 4a collects the exhaust gas discharged from the exhaust gas discharge port 100a of the exhaust pipe 100 and collects the air around the exhaust gas discharge port 100a at the same time.

At this time, the detection sampling part 7a in the exhaust gas collecting part 4 collects the gas in the vicinity of the exhaust gas collecting port 4a and introduces the collected gas into the leak detecting part 7b.

The leak detecting part 7b detects the concentration of the predetermined component contained in the gas introduced from the detection sampling part 7a and outputs this concentration to such as, for example, a monitor. Then, the user compares this outputted concentration to the predetermined threshold and confirms whether or not the exhaust gas leaks.

In the case where the user determines that the exhaust gas is leaking, it is possible for the user to adjust a positional relationship between the exhaust gas discharge port 100a of the exhaust pipe 100 of the internal combustion engine and the exhaust gas collecting port 4a of the exhaust gas collecting part 4 to thereby eliminate the leak of the exhaust gas.

Alternatively, it is possible for the user to decrease the flow rate of the dilution gas flowing in the dilution gas introducing flow passage 6 using a flow adjusting valve 6a while increasing the flow rate of the air to be collected by the exhaust gas collecting part 4 to thereby eliminate the leak of the exhaust gas.

According to the exhaust gas collecting device of the present embodiment configured as described above, the following effects are obtained.

That is, since the exhaust gas detecting mechanism 7 is provided, by confirming the detection result of the exhaust gas detecting mechanism 7, it is possible to detect whether the exhaust gas leaks from a portion between the exhaust pipe 100 and the exhaust gas collecting part 4. Moreover, since the exhaust gas detecting mechanism 7 is provided in the vicinity of the exhaust gas collecting port 4a, in the case of using the open type exhaust gas collecting part 4, it is possible to prevent the exhaust gas from being excessively diluted by the air present around the exhaust pipe 100 and prevent that it becomes difficult for the exhaust gas detecting mechanism 7 to detect the predetermined component contained in the exhaust gas.

Thus, it is possible to accurately perform the exhaust gas analysis using the open type exhaust gas collecting part 4. Further, since it becomes possible to perform the test in conformance with the measuring method defined in such as the Road Vehicle Safety Standard or the international standards, it is possible to ensure reliability for the test result of the exhaust gas test.

Furthermore, since the exhaust gas detecting mechanism 7 is provided for continuously detecting the predetermined component, the leak of the exhaust gas can be detected in real time.

Further, since the detection sampling part 7a for collecting gas in the vicinity of the exhaust gas collecting port 4a of the exhaust gas collecting part 4 includes a plurality of the sampling pipes 70, it is possible to sample the gas at a plurality of points in the vicinity of the exhaust gas collecting port 4a and it is possible to perform the sampling of the gas accurately to detect the leak of the exhaust gas even in the case where s leaking location cannot be determined due to such as vibrations of the exhaust pipe 100.

Moreover, since the multiple sampling tubes 70 are bundled to be a single tube and connected to the leak detecting part 7b, the number of parts can be reduced to simplify the device configuration compared to the case where the leak detecting parts 7b are respectively provided for the multiple sampling tubes 70.

Further, since the taper surface is provided around the connecting portion 4c connected to the main flow passage 5 in the inner peripheral surface of the exhaust gas collecting part 4, even in the case where the exhaust gas and air discharged from the exhaust pipe 100 toward the main flow passage 5 rebound on the taper surface 4b, it is possible to facilitate the exhaust gas and air to flow into the main flow passage 5 without leaking to the outside from the portion between the exhaust gas collecting part 4 and the exhaust pipe 100.

In addition, since the dilution gas flow passage 6 is provided, the flow rate of the air collected from the exhaust gas collecting part 4 can be adjusted by adjusting the flow rate of the dilution gas introduced from the dilution gas flow passage 6. For example, in the case where a user determines that the exhaust gas is leaking, the flow rate of the air to be collected by the exhaust gas collecting part 4 can be increased by decreasing the flow rate of the dilution gas flowing in the dilution gas introducing flow passage 6, and thus the leak of the exhaust gas can be eliminated. Moreover, since the leak of the exhaust gas can be eliminated without changing the flow rate of the fluid flowing in the main flow passage 5 by the constant flow mechanism 9, it is possible to accurately perform the exhaust gas analysis without largely varying a dilution ratio of the exhaust gas.

According to the exhaust gas detecting method of the present embodiment, in the case where the user confirms the concentration of the predetermined substance detected by the exhaust gas detecting mechanism 7 and determines that exhaust gas is leaking, the user can take actions for eliminating the leak of the exhaust gas by adjusting the positional relationship between the exhaust pipe 100 and the exhaust gas collecting part 4 or adjusting the flow rate of the dilution gas flowing in the dilution gas introducing flow passage 6 using the flow adjusting valve 6a. Therefore, the exhaust gas analysis can be accurately performed and it is possible to perform the test in conformance with a measuring method defined in such as the Road Vehicle Safety Standard or the international standards, and it is possible to ensure reliability for the test result of the exhaust gas test.

It is noted that the present invention is not limited to the embodiment described above.

The exhaust gas analysis system of the present embodiment may be configured such that, after collecting a part of the diluted exhaust gas from the main flow passage, the diluted gas is diluted again (two-stage dilution), and the two-stage-diluted exhaust gas is analyzed.

Further, although the exhaust gas collecting device of the present embodiment is configured to collect the exhaust gas discharged from the exhaust pipe of the engine of such as a motorcycle or automobile driven by a chassis dynamometer, it may be configured, for example, to collect the exhaust gas discharged from the exhaust pipe of the engine driven by an engine dynamometer.

In the above embodiment, although the dilution gas introducing flow passage is provided, it is not necessary to provide the dilution gas introducing flow passage. In this case, the air to be mixed with the exhaust gas by the mixing part is only the air collected by the exhaust gas collecting part.

In the above embodiment, although the exhaust pipe is inserted to the exhaust gas collecting port, the exhaust pipe may be disposed so that, for example, the exhaust gas discharge port of the exhaust pipe and the exhaust gas collecting port of the exhaust gas collecting part are disposed so as to be coplanar. Further, the exhaust pipe and the exhaust gas collecting part may be disposed so as to be separated from each other by a predetermined distance.

The exhaust gas detecting mechanism may be configured of: a semiconductor sensor for detecting a concentration of a predetermined component contained in the surrounding gas; and an arithmetic unit receiving a signal detected by the semiconductor sensor and calculating the concentration of the predetermined component which is outputted to such as a monitor. With this configuration, since a delay in time for sending the gas from the sampling tube to the detecting part can be eliminated, the leak of the exhaust gas can be detected still more in real time.

The sampling tube may be installed anywhere so long as a position where the gas in the vicinity of the exhaust gas collecting port can be collected and it may be provided, for example, on the inner peripheral surface of the opening portion of the exhaust gas collecting part. In this case, there may be considered such as a dealing measure that the direction of the opening end portion of the sampling tube is set to be opposite to the flowing direction of the exhaust gas flow so as not to unnecessarily suck the exhaust gas from the exhaust pipe.

Further, the detection sampling part may be configured as follows.

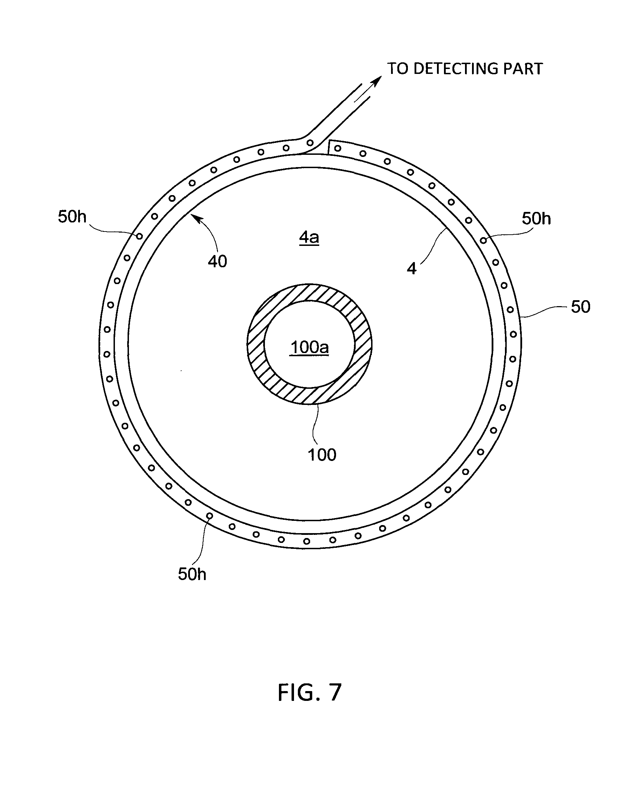

That is, as shown in Fig. 7, the detection sampling part 50 may have one tube provided with multiple holes and this tube may be wound so as to surround an outer peripheral surface of an opening portion 40 of the exhaust gas collecting part 4. With this configuration, since the exhaust gas discharged from the exhaust pipe 100 in the entire part of the opening portion 40 of the exhaust gas collecting part 4 can be collected, the leak of the exhaust gas can be detected more accurately. Moreover, since it is not necessary to provide a plurality of tubes, the detection sampling part 50 can be easily produced with a simple device design.

The analysis equipment connected to the sampling line may be configured of, for example, a PM filter or an exhaust gas analysis device for continuously measuring the collected mixed gas.

In the exhaust gas leak detecting method of the present embodiment, in the case where a main flow passage side has a large negative pressure with respect to an exhaust pipe side of the internal combustion engine, the user also may reduce the negative pressure condition by adjusting the flow rate of the dilution gas using the flow adjusting valve provided in the dilution gas introducing flow passage. Thus, it is possible to reduce a load on the internal combustion engine caused by sucking the exhaust gas more than necessity discharged from the exhaust pipe by the exhaust gas collecting part.

Further, as the gas component detector of the leak detecting part, an existing analysis device capable of appropriately measuring a measurement target component may be also used instead of the exhaust gas analysis device detecting a concentration of the predetermined component by the non-dispersive infrared absorption method. Moreover, since a gas meter like a semiconductor sensor is a compact type, the analysis device can be also directly attached to the outer peripheral surface or inner peripheral surface of the exhaust gas collecting port of the exhaust gas collecting part. Therefore, it becomes possible to detect the leak without providing the sampling tube.

Further, in the case where the concentration of the predetermined component detected by the leak detecting part exceeds the predetermined threshold, it may be configured to draw attention to the user in a manner of, for example, alarming, or informing the user that there is a leak or a possibility of leaking such as displaying a warming screen on the monitor.

Furthermore, in the case where the concentration of the predetermined component detected by the leak detecting part exceeds the predetermined threshold, it may be configured so as to automatically adjust the positional relationship between the exhaust gas discharge port of the exhaust pipe of the internal combustion engine and the exhaust gas collecting port of the exhaust gas collecting part. Alternatively, it may be also configured so as to adjust the flow rate of the dilution gas by automatically adjusting a valve opening degree of the flow adjusting valve provided on the dilution gas introducing flow passage.

In addition, in the above embodiment, although the entire part of the inner peripheral surface of the exhaust gas collecting part is formed as a taper surface, a partial portion in the direction of the flow passage in the inner peripheral surface of the exhaust gas collecting part may be formed as a taper surface so long as an aperture size of the exhaust gas collecting port is throttled to an aperture size in cross section of the main flow passage.

Further, the opening shape of the exhaust gas collecting port is not limited to a circular shape, but, for example, a polygonal shape such as a rectangular or triangular shape or ellipse shape may be also used.

The constant flow mechanism is not limited to a configuration composed of the critical flow venturi and the suction pump, but the other various devices such as a critical orifice and a suction blower or a positive displacement pump type CVS device (positive displacement pump: PDP) etc. may be used. Further, a variable flow control mechanism, that is, a flow control mechanism capable of variably controlling the flow rate of the fluid flowing in the main flow passage may be used instead of the constant flow mechanism.

In addition, the mixing part may be provided or omitted.

The present invention is not limited to the above embodiments and various modifications thereof can be made in a range without departing from the spirit thereof.

### Reference Signs List

2 ··· Exhaust gas collecting device
4 ··· Exhaust gas collecting part
4a ··· Exhaust gas collecting port
5 ··· Main flow passage
7 ··· Exhaust gas detecting mechanism
100 ··· Exhaust pipe
100a ··· Exhaust gas discharge port

## Claims

1. An exhaust gas analysis system comprising: an exhaust gas collecting device for collecting exhaust gas from an exhaust pipe in which the exhaust gas discharged from an internal combustion engine flows; and gas analysis equipment for analyzing the exhaust gas collected by the exhaust gas collecting device, wherein
the exhaust gas collecting device comprises:
an exhaust gas collecting part which is provided at an exhaust gas discharge port of the exhaust pipe, having an exhaust gas collecting port that is larger than the exhaust gas discharge port, for collecting air around the exhaust gas discharge port together with the exhaust gas discharged from the exhaust gas discharge port;
a main flow passage which is connected to the exhaust gas collecting part and in which the exhaust gas and the air collected from the exhaust gas collecting part flow; and
an exhaust gas detecting mechanism for continuously detecting a predetermined component contained in the exhaust gas in the vicinity of the exhaust gas collecting port in the exhaust gas collecting part.

2. The exhaust gas analysis system according to claim 1, wherein the exhaust gas detecting mechanism includes: a detection sampling part for collecting gas in the vicinity of the exhaust gas collecting port of the exhaust gas collecting part; and a leak detecting part for detecting a concentration of the predetermined component contained in the exhaust gas in the gas collected by the detection sampling part, and wherein
the detection sampling part includes a plurality of sampling pipes in the vicinity of the exhaust gas collecting port.

3. The exhaust gas analysis system according to claim 1, wherein the exhaust gas detecting mechanism is adapted to measure carbon dioxide or carbon monoxide.

4. The exhaust gas analysis system according to claim 1, wherein the exhaust gas collecting part has a taper surface with an opening in cross section thereof gradually decreases toward a side of a connecting portion of the main flow passage from a side of the exhaust gas collecting port in an inner peripheral surface of the exhaust gas collecting part.

5. The exhaust gas analysis system according to claim 1 further comprising a constant flow mechanism provided on the main flow passage for maintaining a flow rate of fluid flowing in the main flow passage constant.

6. The exhaust gas analysis system according to claim 5 further comprising a dilution gas flow passage provided on the main flow passage and connected to an upstream side of the constant flow mechanism for introducing dilution gas into the main flow passage.

7. The exhaust gas analysis system according to claim 1 further comprising a leak detecting part for comparing a detection value detected by the exhaust gas detecting mechanism with a predetermined threshold value to thereby determine whether the exhaust gas leaks from the exhaust gas collecting port and outputting a warning in the case where the exhaust gas is leaking.

8. An exhaust gas leak detecting method of an exhaust gas analysis system comprising exhaust gas analysis equipment for analyzing exhaust gas collected by an exhaust gas collecting device for collecting exhaust gas from an exhaust pipe in which the exhaust gas discharged from an internal combustion engine flows, wherein
the exhaust gas collecting device comprises:
an exhaust gas collecting part which is provided at an exhaust gas discharge port of the exhaust pipe in which the exhaust gas flows and has an exhaust gas collecting port that is larger than the exhaust gas discharge port, and
an exhaust gas detecting mechanism for detecting a predetermined component contained in the exhaust gas in the vicinity of the exhaust gas collecting port in the exhaust gas collecting part, thereby detecting a leak of the exhaust gas using detection results detected by the exhaust gas detecting mechanism.
